# EUROPEAN PATENT APPLICATION

(11) **EP 4 212 167 A1**
(43) Date of publication of application: **19.07.2023**
(21) Application number: 21862072.2
(22) Date of filing: 25.08.2021
(51) Int. Cl.: A61K 38/08, A61P 25/28, A23L 33/18

(54) **PEPTIDE COMPOSITION FOR PREVENTION OR TREATMENT OF ALZHEIMER'S DISEASE**

(30) Priority: 27.08.2020 KR 20200108861
(71) Applicant: HLB Science Inc., Seongdong-gu Seoul 04782 (KR)
(72) Inventor: PARK, Yeong Min, Seoul 03356 (KR); CHOI, Wahn Soo, Seoul 06325 (KR); LEE, Seung-Hyun, Seoul 05229 (KR); JUNG, In Duk, Cheongju-si, Chungcheongbuk-do 28410 (KR); KIM, Yong Joo, Hwaseong-si, Gyeonggi-do 18470 (KR); LEE, Seung Jun, Yeongju-si, Gyeongsangbuk-do 36002 (KR); KIM, Sung Min, Sejong-si 30100 (KR); LEE, Mi Suk, Seoul 08358 (KR); PARK, Hee Jo, Seoul 05022 (KR); CHOI, Seung Pyo, Seoul 05007 (KR); MOON, Minho, Daejeon 35356 (KR); SHIN, Soo Jung, Daejeon 34070 (KR); KIM, Sujin, Daejeon 35366 (KR); PARK, Yong Ho, Daejeon 35360 (KR); PARK, Jae-Yong, Seoul 02799 (KR); LEE, Kun Ho, Gwangju 61746 (KR)
(74) Representative: Dehns
(86) International application number: PCT/KR2021/011386
(87) International publication number: WO 2022/045775

(57) **Abstract**

The present invention relates to a peptide composition for preventing or treating Alzheimer's dementia. A peptide or a salt substituent thereof according to the present invention exhibits effects such as suppression of LPS-mediated cytokine production, suppression of LPS-induced neuroinflammation, amelioration of cognitive impairment, suppression of beta amyloid or tau protein aggregation, and suppression of neuronal loss. The polypeptide or the salt substituent thereof can permeate the blood-brain barrier, and thus, is expected to be usefully used for preventing or treating Alzheimer's dementia.

## Description

### [Technical Field]

The present invention relates to a peptide composition for preventing or treating Alzheimer's dementia.

This application claims priority to and the benefit of Korean Patent Application No. 10-2020-0108861 filed in the Korean Intellectual Property Office on August 27, 2020, and all the contents disclosed in the specification and drawings of that application are incorporated in this application.

### [Background Art]

Dementia is a pathological phenomenon that should be distinguished from normal aging, and is classified into Alzheimer's dementia, vascular dementia, and other types of dementia due to alcoholism, trauma, or sequelae of Parkinson's disease according to its cause.

Among them, the pathogenesis of Alzheimer's dementia is not clearly known, but the toxicity of beta-amyloid, which is a neurotoxic protein, has been proposed as an important cause. This material is known to be produced by the incorrect metabolism of an amyloid precursor protein (APP), and the normal metabolite of the APP has been reported to have a neuroprotective effect. Recent studies using a genetic engineering method have revealed that toxic proteins, such as β-amyloid, accumulate in cells and blood vessels, which is toxic to nerve cells, resulting in impaired brain function.

Although the number of patients with Alzheimer's dementia is significantly increasing due to the aging population and the disease is rapidly emerging as a social problem to be solved, there are almost no food raw materials or drugs capable of effectively preventing or treating the disease. Dementia is a neurological disease that afflicts 10% or more of the elderly population aged 65 and over, 50% or more of dementia is reported to be Alzheimer's dementia, and in 2017, the elderly population proportion exceeded 7 million, which is 14% of the total population, and is expected to rapidly increase to 41.0% in 2060. Further, the number of dementia patients has reached 724,000 as of 2017, and is expected to increase to 1.68 million in 2040 and 2.127 million or more in 2050 as the population ages.

However, the current therapeutic methods for dementia are measures not for solving the fundamental cause, but merely for the purpose of alleviating the symptoms, and there is a need for developing a material for suppressing, delaying, or treating the onset of dementia.

### [Disclosure]

### [Technical Problem]

As a result of research for the development of agents for preventing or treating Alzheimer's dementia, the present inventors developed a peptide that exhibits effects such as suppression of lipopolysaccharide (LPS)-mediated cytokine production, suppression of neuroinflammation, amelioration of cognitive impairment, suppression of beta-amyloid or tau protein, and suppression of neuronal loss, thereby completing the present invention based on this.

Therefore, an object of the present invention is to provide a peptide composition for preventing, treating or ameliorating Alzheimer's dementia.

However, the technical problems which the present invention intends to solve are not limited to the technical problems that have been mentioned above, and other technical problems which have not been mentioned will be clearly understood by a person with ordinary skill in the art to which the present invention pertains from the following description.

### [Technical Solution]

To achieve the aforementioned object, the present invention provides a pharmaceutical composition for preventing or treating Alzheimer's dementia, including a polypeptide represented by the following sequence general formula or a salt substitute thereof as an active ingredient:

[General Formula] Ln-X1-L-X2-V-X3-X4-X5-R-X6-L-X7

In General Formula,
n is 0 or 1;
L is leucine;
V is valine;
R is arginine;
X1 is lysine (K) or arginine (R);
X2 is glycine (G) or arginine (R);
X3 is glutamic acid (E) or lysine (K);
X4 is alanine (A) or leucine (L);
X5 is lysine (K), arginine (R) or leucine (L);
X6 is tyrosine (Y), alanine (A), tryptophan (W), lysine (K) or aspartic acid (D); and
X7 is aspartic acid (D) or arginine (R),
provided that in General Formula, a polypeptide represented by a sequence of K-L-G-V-E-A-K-R-Y-L-D is excluded.

Further, the present invention provides a food composition for preventing or ameliorating Alzheimer's dementia, including a polypeptide represented by the following sequence general formula or a salt substitute thereof as an active ingredient:

[General Formula] Ln-X1-L-X2-V-X3-X4-X5-R-X6-L-X7

In General Formula,
n is 0 or 1;
L is leucine;
V is valine;
R is arginine;
X1 is lysine (K) or arginine (R);
X2 is glycine (G) or arginine (R);
X3 is glutamic acid (E) or lysine (K);
X4 is alanine (A) or leucine (L);
X5 is lysine (K), arginine (R) or leucine (L);
X6 is tyrosine (Y), alanine (A), tryptophan (W), lysine (K) or aspartic acid (D); and
X7 is aspartic acid (D) or arginine (R),
provided that in General Formula, a polypeptide represented by a sequence of K-L-G-V-E-A-K-R-Y-L-D is excluded.

As an exemplary embodiment of the present invention, the food composition may be a health functional food composition, but is not limited thereto.

As an exemplary embodiment of the present invention, the polypeptide may be a polypeptide consisting of the following amino acids, but is not limited thereto:
in General Formula,
n is 0;
X1 is lysine (K);
X2 is arginine (R);
X3 is lysine (K);
X4 is leucine (L);
X5 is arginine (R);
X6 is tyrosine (Y), and
X7 is arginine (R).

As another exemplary embodiment of the present invention, the polypeptide may be an L-form, a D-form, a peptidomimetic including a peptoid, or a non-natural amino acid, but is not limited thereto.

As still another exemplary embodiment of the present invention, the end of the polypeptide may be alkylated, PEGylated, or amidated, but is not limited thereto.

As yet another exemplary embodiment of the present invention, the polypeptide may have an amine group (NH₂) added to the C-terminus, but is not limited thereto.

As yet another exemplary embodiment of the present invention, the salt substitute of the polypeptide may be an acetate salt substitute, but is not limited thereto.

As yet another exemplary embodiment of the present invention, the polypeptide or the salt substitute thereof may have one or more characteristics of the following characteristics, but is not limited thereto:
suppression of lipopolysaccharide (LPS)-mediated cytokine production;
suppression of neuroinflammation;
amelioration of cognitive impairment;
suppression of beta-amyloid or tau protein aggregation; and
suppression of neuronal loss.

As yet another exemplary embodiment of the present invention, the polypeptide or the salt substitute thereof may pass through the blood brain barrier, but is not limited thereto.

Further, the present invention provides a method for preventing or treating Alzheimer's dementia, the method including administering a composition including the polypeptide or the salt substitute thereof as an active ingredient to a subject in need.

In addition, the present invention provides a use of a composition including the polypeptide or the salt substitute thereof as an active ingredient for preventing or treating Alzheimer's dementia.

Furthermore, the present invention provides a use of the polypeptide or the salt substitute thereof for preparing a drug for treating Alzheimer's dementia.

### [Advantageous Effects]

A peptide or a salt substituent thereof according to the present invention exhibits effects such as suppression of LPS-mediated cytokine production, suppression of LPS-induced neuroinflammation, amelioration of cognitive impairment, suppression of beta amyloid or tau protein aggregation, and suppression of neuronal loss. The polypeptide or the salt substituent thereof can pass through the blood-brain barrier, and thus is expected to be usefully used for preventing or treating Alzheimer's dementia.

### [Description of Drawings]

FIG. 1A is a set of views illustrating the results of confirming the LPS(+) region in the cerebral hemispheres of mice with Alzheimer's dementia according to an exemplary embodiment of the present invention by immunohistochemical staining.
FIG. 1B is a view illustrating the results of confirming the expression of LPS in the secondary motor cortex (M2) of the brain of the mouse with Alzheimer's dementia according to an exemplary embodiment of the present invention by immunohistochemical staining.
FIG. 1C is a view illustrating the results of confirming the expression of LPS in the hippocampus of the Alzheimer's dementia mouse according to an exemplary embodiment of the present invention by immunohistochemical staining.
FIG. 2 is a view confirming LPS levels in blood and cerebrospinal fluid (CSF) samples of Alzheimer's dementia patients by clinical stage according to an exemplary embodiment of the present invention.
FIG. 3 is a view confirming the mRNA expression of IL-1β and TNF-α by administration of a DD-S052 peptide according to an exemplary embodiment of the present invention.
FIG. 4A is a view illustrating an experimental process for confirming the effect of the DD-S052 peptide on LPS-induced neuroinflammation in the brains of wild-type mice according to an exemplary embodiment of the present invention.
FIG. 4B is a view illustrating the results of performing immunohistochemical staining on Iba-1 in order to confirm microglia in the hippocampus of the wild-type mouse according to an exemplary embodiment of the present invention.
FIG. 4C is a view illustrating the quantification of the number of Iba-1 positive cells in in the hippocampus of wild-type mice according to an exemplary embodiment of the present invention.
FIG. 4D is a view illustrating the results of performing immunohistochemical staining on Iba-1 to confirm microglia in the substantia nigra pars compacta of the wild-type mouse brain according to an exemplary embodiment of the present invention.
FIG. 5A is a view illustrating the experimental process for confirming the effect of the DD-S052 peptide on LPS-induced neuroinflammation in the brains of Alzheimer's dementia mice according to an exemplary embodiment of the present invention.
FIG. 5B is a view illustrating the results of performing immunohistochemical staining on Iba-1 in order to confirm microglia in the brains of the Alzheimer's dementia mouse according to an exemplary embodiment of the present invention.
FIG. 6A is a view illustrating an experimental process for confirming the effect of the DD-S052 peptide on cognitive impairment and pathology related to Alzheimer's dementia in mice with Alzheimer's dementia according to an exemplary embodiment of the present invention.
FIG. 6B is a set of views confirming a spontaneous alternation (left view) and a total arm entry (right view) by administration of the DD-S052 peptide in mice with Alzheimer's dementia according to an exemplary embodiment of the present invention.
FIG. 6C is a set of views confirming the effect of suppressing beta-amyloid accumulation by administration of the DD-S052 peptide in mice with Alzheimer's dementia according to an exemplary embodiment of the present invention.
FIG. 6D is a set of views confirming the effect of suppressing microgliosis by administration of the DD-S052 peptide in mice with Alzheimer's dementia according to an exemplary embodiment of the present invention.
FIG. 6E is a set of views confirming the effect of suppressing neuronal loss by administration of the DD-S052 peptide in mice with Alzheimer's dementia according to an exemplary embodiment of the present invention.
FIG. 7 is a set of views confirming the effect of suppressing tau protein aggregation by DD-S052 and PEGylated DD-S052 peptide administration in mice with Alzheimer's dementia according to an exemplary embodiment of the present invention.
FIG. 8A is a view illustrating the results of analyzing the DD-S052 peptide according to an exemplary embodiment of the present invention by liquid-chromatography-mass spectrometry.
FIG. 8B is a set of views illustrating the results of analyzing an artificial membrane permeation test using the DD-S052 peptide according to an embodiment of the present invention by liquid-chromatography-mass spectrometry.
FIG. 8C is a view illustrating the degree of vascular-brain barrier penetration of DD-S052 according to an exemplary embodiment of the present invention.

### [Modes of the Invention]

The present invention provides a pharmaceutical composition for preventing or treating Alzheimer's dementia, including a polypeptide represented by the following sequence general formula or a salt substitute thereof as an active ingredient:

[General Formula] Ln-X1-L-X2-V X3-X4-X5-R-X6-L-X7

In General Formula,
n is 0 or 1;
L is leucine;
V is valine;
R is arginine;
X1 is lysine (K) or arginine (R);
X2 is glycine (G) or arginine (R);
X3 is glutamic acid (E) or lysine (K);
X4 is alanine (A) or leucine (L);
X5 is lysine (K), arginine (R) or leucine (L);
X6 is tyrosine (Y), alanine (A), tryptophan (W), lysine (K) or aspartic acid (D); and
X7 is aspartic acid (D) or arginine (R),
provided that in General Formula, a polypeptide represented by a sequence of K-L-G-V-E-A-K-R-Y-L-D is excluded.

Further, the present invention provides a food composition for preventing or ameliorating Alzheimer's dementia, including the polypeptide represented by the aforementioned sequence general formula or a salt substitute thereof as an active ingredient.

Further, the present invention provides a method for preventing or treating Alzheimer's dementia, the method including administering a composition including the polypeptide or the salt substitute thereof as an active ingredient to a subject in need.

In addition, the present invention provides a use of a composition including the polypeptide or the salt substitute thereof as an active ingredient for preventing or treating Alzheimer's dementia.

Furthermore, the present invention provides a use of the polypeptide or the salt substitute thereof for preparing a drug for treating Alzheimer's dementia.

According to an exemplary embodiment of the present invention, the polypeptide may be a polypeptide consisting of the following amino acids, but is not limited thereto.
in General Formula,
n is 0;
X1 is lysine (K);
X2 is arginine (R);
X3 is lysine (K);
X4 is leucine (L);
X5 is arginine (R);
X6 is tyrosine (Y), and
X7 is arginine (R).

As used herein, "polypeptide" refers to a linear molecule formed by bonding amino acid residues to each other by peptide bonds. The polypeptide of the present invention may be prepared by a chemical synthesis method known in the art (for example, solid-phase synthesis techniques) along with molecular biological methods (Merrifield, J. Amer. Chem. Soc. 85: 2149-54(1963); Stewart, et al., Solid Phase Peptide Synthesis, 2nd. ed., Pierce Chem. Co.: Rockford, 111(1984)).

Further, the scope of the polypeptide of the present invention may include biologically functional equivalents with variations in the amino acid sequence that exert a biological activity equivalent to the polypeptide of the present invention. Such variations in the amino acid sequence may be based on the relative similarity of side chain substituents of amino acids in terms of aspects such as hydrophobicity, hydrophilicity, charge and size. An analysis of the size, shape and type of amino acid side chain substitute revealed that arginine, lysine and histidine are all positively charged residues; alanine, glycine and serine have similar sizes; and phenylalanine, tryptophan and tyrosine have similar shapes. Therefore, based on these considerations, arginine, lysine and histidine; alanine, glycine and serine; and phenylalanine, tryptophan and tyrosine can be said to be biologically functional equivalents.

In introducing the variation, the hydrophobicity index of the amino acid may be considered. Each amino acid is assigned a hydrophobicity index, according to its hydrophobicity and charge. Further, it is also known that substitutions between amino acids with similar hydrophilicity values result in peptides with equivalent biological activity.

Amino acid exchanges in peptides that do not totally alter the activity of the molecule are known in the art (H. Neurath, R.L.Hill, The Proteins, Academic Press, New York, 1979). The most typically occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly.

In consideration of the variations having the above-described biologically equivalent activity, the polypeptide of the present invention is also interpreted to include a sequence showing substantial identity. The aforementioned substantial identity may refer to a random sequence differing from the sequence of the present invention and having at least 80% homology, 90% homology, or 95% homology to the sequence of the present invention, when it is aligned to correspond to the sequence of the present invention as much as possible, and the aligned sequences are analyzed using an algorithm typically used in the art. Alignment methods for sequence comparison are known in the art (Huang et al., Comp. Appl. BioSci. 8:155-65(1992); Pearson et al., Meth. Mol. Biol. 24:307-31(1994)).

In an exemplary embodiment of the present invention, the polypeptide may be an L-form, a D-form, a peptidomimetic including a peptoid, or a non-natural amino acid.

The D-form polypeptide refers to an allomeric type polypeptide as an enantiomer having the same amino acid sequence as the L-form polypeptide.

In an exemplary embodiment of the present invention, the polypeptide represented by the sequence general formula according to the present invention may have an amine group (NH₂) added to the C-terminus, but is not limited thereto.

Adding an amine group (NH₂) to the C-terminus of a polypeptide may follow various methods known in the art.

In addition, a salt (or a salt substitute) thereof may be included within the scope of the polypeptide of the present invention.

The salt includes, for example, acid addition salts formed by free acids and pharmaceutically acceptable metal salts.

Examples of a suitable acid include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, gluconic acid, naphthalene-2-sulfonic acid, benzenesulfonic acid, and the like. An acid addition salt may be prepared by a typical method, for example, dissolving a compound in an excessive amount of an aqueous acid solution and precipitating the salt using a water-miscible organic solvent such as methanol, ethanol, acetone or acetonitrile. In addition, the acid addition salt may be prepared by heating an equimolar amount of compound and an acid or alcohol in water, and then evaporating the mixture to dry the mixture, or suction-filtering the precipitated salt.

A salt derived from a suitable base may include an alkali metal such as sodium and potassium, an alkaline earth metal such as magnesium, ammonium and the like, but is not limited thereto. An alkali metal or alkaline earth metal salt may be obtained by, for example, dissolving the compound in an excessive amount of an alkali metal hydroxide or alkaline earth metal hydroxide solution, filtering a non-soluble compound salt, evaporating the filtrate, and drying the resulting product. In this case, it is particularly suitable to prepare a sodium, potassium or calcium salt as the metal salt from the pharmaceutical perspective, and the corresponding silver salt may also be obtained by reacting an alkali metal or alkaline earth metal salt with a suitable silver salt (for example, silver nitrate).

As part of the salts of the above-described polypeptide, the present invention provides acetate salts of the polypeptide.

More specifically, provided is an acetate salt substitute of trifluoroacetic acid (TFA) of the polypeptide according to the present invention.

According to an exemplary embodiment of the present invention, the peptide (KLRVKLRRYLR-NH₂ (AcOH), SEQ ID NO: 1, hereinafter referred to as DD-S052) of the present invention is provided as an allomeric type polypeptide having the same amino acid sequence as FP13-NH₂ (SEQ ID NO: 5) designed based on an FP3 and LPS binding model to enhance the interaction with LPS in the residue sequence of FP3 (SEQ ID NO: 4) with a point mutation in FP1 (SEQ ID NO: 3), and in the peptide DD-S052 of the present invention, (AcOH) means that trifluoroacetic acid (TFA) at the ninth amino acid from the N-terminus of the peptide was substituted with an acetate salt (see Example 2).

In the present invention, the end of the polypeptide represented by the sequence general formula according to the present invention may be alkylated, PEGylated, or amidated, but is not limited thereto.

In an exemplary embodiment of the present invention, DD-S052P (SEQ ID NO: 2) is provided by PEGylating the N-terminus of DD-S052 (SEQ ID NO: 1) in the polypeptide according to the invention. Although not limited thereto, the polypeptide according to the present invention may be reacted with Fmoc-NH-PEG2-CH₂COOH for the PEGylation, and in this case, polyethylene glycol has a molecular weight (Mw) of 385.4 Da.

Those skilled in the art may adjust the conditions for PEGylation according to the selected polypeptide, and the method of PEGylation may follow various methods known in the art.

For alkylation or amidation, those skilled in the art may also adjust the conditions for alkylation or amidation according to the selected polypeptide, and the method of alkylation or amidation may follow various methods known in the art.

As described above, the present invention provides: the polypeptide represented by the sequence general formula; a polypeptide which is an L-form, a D-form, a peptidomimetic including a peptoid, or a non-natural amino acid; an acetate salt of a polypeptide having an amine group (NH₂) added to the C-terminus; or a polypeptide whose end is alkylated, PEGylated or amidated.

In the present invention, the polypeptide or the salt substitute thereof may have one or more characteristics of the following characteristics, but is not limited thereto:
suppression of lipopolysaccharide (LPS)-mediated cytokine production;
suppression of neuroinflammation;
amelioration of cognitive impairment;
suppression of beta-amyloid or tau protein aggregation; and
suppression of neuronal loss.

In an exemplary embodiment of the present invention, it was confirmed that lipopolysaccharide (LPS)-positive cells increased with age in the brain of an Alzheimer's dementia mouse model, and it was confirmed that LPS was expressed in microglia in 3- and 5.5-month-old mice, and in neurons in 11-month-old mice. In addition, it was confirmed that LPS(+) cells are abundantly expressed in the secondary motor cortex of the brain and are mainly distributed in the polymorph layer of the dentate gyrus in the hippocampus (see Example 1).

In an exemplary embodiment of the present invention, as a result of confirming the LPS levels in blood and cerebrospinal fluid (CSF) samples of patients with Alzheimer's dementia by clinical stage, it was confirmed that a higher LPS level was measured as Alzheimer's dementia progressed (see Example 2).

In an exemplary embodiment of the present invention, the following characteristics of the polypeptide according to the invention were confirmed, but are not limited to these characteristics.

In an exemplary embodiment of the present invention, it was confirmed that the polypeptide according to the present invention suppresses the mRNA expression of cytokines IL-1β and TNF-α induced by lipopolysaccharide (LPS) (see Example 4).

In an exemplary embodiment of the present invention, it was confirmed by an immunohistochemical method that the polypeptide according to the present invention suppressed LPS-induced microgliosis in the hippocampus and substantia nigra pars compacta of the wild-type mouse brain, thereby exhibiting an inhibitory effect on neuroinflammation (see Example 5).

In an exemplary embodiment of the present invention, it was confirmed by an immunohistochemical method that the polypeptide according to the present invention exhibited an inhibitory effect on neuroinflammation induced by LPS in the brains of mice with Alzheimer's dementia (see Example 6).

In an exemplary embodiment of the present invention, it was confirmed by cell immunofluorescence staining that the polypeptide according to the present invention ameliorated cognitive impairment and suppressed Alzheimer's dementia-related pathologies such as beta-amyloid accumulation, microgliosis, and neuronal loss (see Example 7).

In an exemplary embodiment of the present invention, it was confirmed that the polypeptide and PEGylated polypeptide according to the present invention exhibit tau protein aggregation suppression effects (see Example 8).

In an exemplary embodiment of the present invention, it was confirmed that the polypeptide according to the present invention penetrates the blood-brain barrier, and the permeability at this time corresponds to the median value (see Example 9).

In the present invention, "Alzheimer's dementia" is a degenerative brain disease that is characterized by gradual memory loss and cognitive impairment mainly in elderly people aged 65 and over. At the pathological level, the entanglement of beta-amyloid protein deposits and nerve fiber tau proteins is recognized as a typical etiology. Alzheimer's dementia is often accompanied by psychobehavioral symptoms such as personality changes, agitation, depression, delusions, hallucinations, increased aggression, and sleep disorders, as well as cognitive decline during its progression, and at the end of Alzheimer's dementia, neurological disorders such as stiffness and gait abnormalities, as well as physical complications such as bowel and bladder incontinence, infections, and bedsores appear. When the brain tissue of Alzheimer's dementia patients is examined under a microscope, characteristic lesions such as neuritic plaques and neurofibrillary tangles are observed, and overall brain atrophy due to neuronal loss is seen during visual observation. Such brain pathological findings are mainly limited to the hippocampus and entorhinal cortex regions, which are the main brain regions responsible for memory, in the early stage of the disease, but gradually spread to the entire brain via the parietal lobe, frontal lobe, and the like. According to the progression of involved cerebral pathological sites, memory loss mainly appears in the early stage, and as Alzheimer's dementia progresses, clinical symptoms vary and become more severe while showing a gradual course.

As used herein, "microglia" refers to cells that perform primary immune functions in the central nervous system, and when toxins introduced from the outside or generated inside are present while maintaining the shapes of elongated branches and thin cell bodies, microglia are transformed into an activated shape having thick and short branches and thick cell bodies in order to protect nerve cells from these toxins Unlike microglia in a normal state, activated microglia activate phagocytosis, proliferate cells, and produce inflammation mediators by expressing genes such as cytokines such as TNF-α, IL-1β and IL-6, chemokines, inducible nitric oxide synthase (iNOS) and cyclooxygenase-2 (COX-2). Although the activation of microglia has one side of removing damaged cells and protecting nerve cells from invading bacteria and viruses from the outside, there is another side of nitric oxide produced by iNOS and prostaglandins produced by COX-2, TNF-α, and the like exhibiting toxicity to nerve cells, so the activation of microglia exacerbates the damage to nerve cells.

In the present invention, "neuroinflammation" includes a wide range of complex cellular responses involving the activation of microglia and astrocytes and cytokines, chemokines, complement proteins, acute-phase proteins, oxidative damage and related molecular processes, and the events may adversely affect neuronal function to cause neuronal damage, additional glial activation, and ultimately neurodegeneration.

In the present invention, "lipopolysaccharide (LPS)" consists of lipid A, the core polysaccharide, an O-specific polysaccharide side chain. Lipopolysaccharide is thermally stable and exhibits strong antigenicity. Lipopolysaccharide forms a plurality of complexes with a basic unit with a molecular weight of 10 kDa, varying in size up to 10,000 kDa, and is chemically an amphiphilic complex. Lipopolysaccharide causes inflammatory responses in animals and is mostly present in the outer membrane of Gram-negative bacteria such as Salmonella and *E. coli.* Among lipopolysaccharides, a toxic portion that causes such inflammatory responses is the lipid portion, and is called endotoxin. When such endotoxins enter the human body, fever, changes in white blood cell count, vascular coagulation, tumor necrosis, hypotension, shock, and the like occur, and in addition, endotoxin causes the production of various cytokines such as IL-1, IL-6, IL-8 and TNF, the activation of immune system complements, the activation of blood coagulation, and the like, and acts as a mitogen for B cells to increase polyclonal differentiation and B cell proliferation, and IgM and IgG antibody production.

The pharmaceutical composition of the present invention may further include an appropriate carrier, excipient, and diluent, which are typically used to prepare a pharmaceutical composition. The excipient may be, for example, one or more selected from the group consisting of a diluent, a binder, a disintegrant, a lubricant, an adsorbent, a moisturizer, a film-coating material, and a controlled release additive.

The pharmaceutical composition according to the present invention may be used by being formulated into the form of an external preparation such as a powder, a granule, a sustained-release granule, an enteric granule, a liquid, a collyrium, an elixir, an emulsion, a suspension, a spirit, a troche, aromatic water, a limonade, a tablet, a sustained-release tablet, an enteric tablet, a sublingual tablet, a hard capsule, a soft capsule, a sustained-release capsule, an enteric capsule, a pill, a tincture, a soft extract agent, a dry extract agent, a fluid extract agent, an injection, a capsule, a perfusate, a plaster, a lotion, a paste, a spray, an inhalant, a patch, a sterilized injection solution, or an aerosol, and the external preparation may have a formulation such as a cream, a gel, a patch, a spray, an ointment, a plaster, a lotion, a liniment, a paste or a cataplasma.

Examples of a carrier, an excipient or a diluent which may be included in the composition according to the present invention include lactose, dextrose, sucrose, an oligosaccharide, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

When the pharmaceutical composition is prepared, the pharmaceutical composition is prepared using a diluent or excipient, such as a filler, an extender, a binder, a wetting agent, a disintegrant, and a surfactant, which are commonly used.

As an additive of the tablet, powder, granule, capsule, pill, and troche according to the present invention, it is possible to use an excipient such as corn starch, potato starch, wheat starch, lactose, sucrose, glucose, fructose, D-mannitol, precipitated calcium carbonate, synthetic aluminum silicate, calcium monohydrogen phosphate, calcium sulfate, sodium chloride, sodium hydrogen carbonate, purified lanolin, microcrystalline cellulose, dextrin, sodium alginate, methyl cellulose, carboxymethyl cellulose sodium, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and Primojel; and a binder such as gelatin, arabic gum, ethanol, agar powder, cellulose acetate phthalate, carboxymethyl cellulose, carboxymethyl cellulose calcium, glucose, purified water, sodium caseinate, glycerin, stearic acid, carboxymethyl cellulose sodium, methylcellulose sodium, methylcellulose, microcrystalline cellulose, dextrin, hydroxycellulose, hydroxypropyl starch, hydroxymethyl cellulose, purified shellac, starch, hydroxypropyl cellulose, hydroxypropyl methyl cellulose, polyvinyl alcohol, and polyvinylpyrrolidone, and it is possible to use a disintegrant such as hydroxypropyl methyl cellulose, corn starch, agar powder, methyl cellulose, bentonite, hydroxypropyl starch, carboxymethyl cellulose sodium, sodium alginate, carboxymethyl cellulose calcium, calcium citrate, sodium lauryl sulfate, silicic anhydride, 1-hydroxypropyl cellulose, dextran, an ion exchange resin, polyvinyl acetate, formaldehyde-treated casein and gelatin, alginic acid, amylose, guar gum, sodium bicarbonate, polyvinylpyrrolidone, calcium phosphate, gelled starch, arabic gum, amylopectin, pectin, sodium polyphosphate, ethyl cellulose, sucrose, magnesium aluminum silicate, a D-sorbitol solution, and light anhydrous silicic acid; and a lubricant such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubriwax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid.

As an additive for liquid formulation according to the present invention, it is possible to use water, diluted hydrochloric acid, diluted sulfuric acid, sodium citrate, sucrose monostearate, polyoxyethylene sorbitol fatty acid esters (Tween esters), polyoxyethylene monoalkyl ether, lanolin ether, lanolin esters, acetic acid, hydrochloric acid, aqueous ammonia, ammonium carbonate, potassium hydroxide, sodium hydroxide, prolamin, polyvinyl pyrrolidone, ethyl cellulose, carboxymethyl cellulose sodium, and the like.

In a syrup according to the present invention, a solution of sucrose, other sugars or sweeteners, and the like may be used, and a fragrance, a colorant, a preservative, a stabilizer, a suspending agent, an emulsifier, a thickener, and the like may be used, if necessary.

Purified water may be used for the emulsion according to the present invention, and an emulsifier, a preservative, a stabilizer, a fragrance, and the like may be used, if necessary.

In the suspending agent according to the present invention, a suspending agent such as acacia, tragacanth, methyl cellulose, carboxymethyl cellulose, carboxymethyl cellulose sodium, microcrystalline cellulose, sodium alginate, hydroxypropyl methyl cellulose (HPMC), HPMC 1828, HPMC 2906, and HPMC 2910 may be used, and a surfactant, a preservative, a colorant, and a fragrance may be used, if necessary.

The injection according to the present invention may include: a solvent such as distilled water for injection, 0.9% sodium chloride injection, Ringer's injection, dextrose injection, dextrose+sodium chloride injection, PEG, lactated Ringer's injection, ethanol, propylene glycol, non-volatile oil-sesame oil, cottonseed oil, peanut oil, corn oil, ethyl oleate, isopropyl myristate, and benzoic acid benzene; a solubilizing aid such as sodium benzoate, sodium salicylate, sodium acetate, urea, urethane, monoethyl acetamide, butazolidin, propylene glycol, Tweens, nijungtinateamide, hexamine, and dimethylacetamide; a buffer such as a weak acid and a salt thereof (acetic acid and sodium acetate), a weak base and a salt thereof (ammonia and ammonium acetate), an organic compound, a protein, albumin, peptone, and gums; an isotonic agent such as sodium chloride; a stabilizer such as sodium bisulfite (NaHSO₃), carbon dioxide gas, sodium metabisulfite (Na₂S₂O₅), sodium sulfite (Na₂SO₃), nitrogen gas (N₂), and ethylenediaminetetraacetic acid; a sulfating agent such as 0.1% sodium bisulfide, sodium formaldehydesulfoxylate, thiourea, disodium ethylenediaminetetraacetate, and acetone sodium bisulfite; an analgesic such as benzyl alcohol, chlorobutanol, procaine hydrochloride, glucose, and calcium gluconate; and a suspending agent such as carboxymethyl cellulose sodium, sodium alginate, Tween 80, and aluminum monostearate.

In a suppository according to the present invention, it is possible to use a base such as cacao butter, lanolin, Witepsol, polyethylene glycol, glycerogelatin, methylcellulose, carboxymethyl cellulose, a mixture of stearic acid and oleic acid, Subanal, cottonseed oil, peanut oil, palm oil, cacao butter+cholesterol, lecithin, ranetwax, glycerol monostearate, Tween or Span, Imhausen, monolen (propylene glycol monostearate), glycerin, Adeps solidus, Buytyrum Tego-G, Cebes Pharma 16, hexalide base 95, Cotomar, Hydroxote SP, S-70-XXA, S-70-XX75(S-70-XX95), Hydrokote 25, Hydrokote 711, idropostal, Massa estrarium (A, AS, B, C, D, E, I, T), Massa-MF, Masupol, Masupol-15, Neosupostal-ene, Paramound-B, Suposhiro (OSI, OSIX, A, B, C, D, H, L), suppository base IV types (AB, B, A, BC, BBG, E, BGF, C, D, 299), Supostal (N, Es), Wecobee (W, R, S, M ,Fs), and tegester triglyceride base (TG-95, MA, 57).

A solid formulation for oral administration includes a tablet, a pill, a powder, a granule, a capsule, and the like, and the solid formulation is prepared by mixing at least one excipient, for example, starch, calcium carbonate, sucrose or lactose, gelatin, and the like with an extract. Further, in addition to a simple excipient, lubricants such as magnesium stearate and talc are also used.

A liquid formulation for oral administration corresponds to a suspension, a liquid for internal use, an emulsion, a syrup, and the like, and the liquid formulation may include, in addition to water and liquid paraffin which are simple commonly used diluents, various excipients, for example, a wetting agent, a sweetener, a fragrance, a preservative, and the like. Examples of a formulation for parenteral administration include an aqueous sterile solution, a non-aqueous solvent, a suspension, an emulsion, a freeze-dried preparation, and a suppository. As the non-aqueous solvent and the suspension, it is possible to use propylene glycol, polyethylene glycol, a vegetable oil such as olive oil, an injectable ester such as ethyl oleate, and the like.

The pharmaceutical composition according to the present invention is administered in a pharmaceutically effective amount. In the present invention, "pharmaceutically effective amount" means an amount sufficient to treat diseases at a reasonable benefit/risk ratio applicable to medical treatment, and an effective dosage level may be determined according to factors including the type of disease of patients, the severity of disease, the activity of drugs, sensitivity to drugs, administration time, administration route, excretion rate, treatment period, and simultaneously used drugs, and other factors well known in the medical field.

The pharmaceutical composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this amount may be easily determined by a person with ordinary skill in the art to which the present invention pertains.

The pharmaceutical composition of the present invention may be administered to a subject in need via various routes. All methods of administration may be expected, but the pharmaceutical composition may be administered by, for example, oral administration, subcutaneous injection, peritoneal administration, intravenous injection, intramuscular injection, paraspinal space (intradural) injection, sublingual administration, buccal administration, intrarectal insertion, intravaginal injection, ocular administration, ear administration, nasal administration, inhalation, spray via the mouth or nose, skin administration, transdermal administration, and the like.

The pharmaceutical composition of the present invention is determined by the type of drug that is an active ingredient, as well as various related factors such as the disease to be treated, the route of administration, the age, sex, and body weight of a patient, and the severity of the disease.

When the polypeptide or the salt substitute thereof of the present invention is used as a food additive, the polypeptide may be added as it is or used with another food or other food ingredients, and may be appropriately used according to a typical method. The amount of active ingredient mixed may be suitably determined according to the purpose of use (prevention, health or therapeutic treatment). In general, when a food or beverage is prepared, the polypeptide of the present invention may be added in an amount of 15 wt% or less, or 10 wt% or less based on the raw materials. However, in the case of long-term intake for the purpose of health and hygiene or for the purpose of controlling health, the amount may be equal to or less than the above range, and the effective ingredient may be used in an amount equal to or more than the above range due to no problem in terms of safety.

The type of food is not particularly limited. Examples of food to which the material may be added include meats, sausage, bread, chocolate, candies, snacks, confectioneries, pizza, instant noodles, other noodles, gums, dairy products including ice creams, various soups, beverages, tea, drinks, alcoholic beverages, vitamin complexes, and the like, and include all health functional foods in a typical sense.

The health beverage composition according to the present invention may contain various flavors or natural carbohydrates, and the like as additional ingredients as in a typical beverage. The above-described natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As a sweetener, it is possible to use a natural sweetener such as thaumatin and stevia extract, a synthetic sweetener such as saccharin and aspartame, and the like. The proportion of the natural carbohydrates is generally about 0.01 to 0.20 g, or about 0.04 to 0.10 g per 100 ml of the composition of the present invention.

In addition to the aforementioned ingredients, the composition of the present invention may contain various nutrients, vitamins, electrolytes, flavors, colorants, pectic acids and salts thereof, alginic acid and salts thereof, organic acids, protective colloid thickeners, pH adjusters, stabilizers, preservatives, glycerin, alcohols, carbonating agents used in carbonated drinks, and the like. In addition, the composition of the present invention may contain flesh for preparing natural fruit juice, fruit juice drinks, and vegetable drinks. These ingredients may be used either alone or in combinations thereof. The proportion of these additives is not very important, but is generally selected within a range of 0.01 to 0.20 part by weight per 100 parts by weight of the composition of the present invention.

As used herein, the subject refers to a subject in need of treatment of Alzheimer's dementia, and more specifically, refers to a mammal such as a human or a non-human primate, a mouse, a rat, a dog, a cat, a horse, and a cow.

The "administration" as used herein refers to the provision of a predetermined composition of the present invention to a subject in need thereof by any suitable method.

As used herein, the "prevention" refers to all actions that suppress or delay the onset of Alzheimer's dementia, and the "treatment" refers to all actions that ameliorate or beneficially change Alzheimer's dementia by administration of the pharmaceutical composition according to the present invention, and the "amelioration" refers to all actions that reduce Alzheimer's dementia and associated parameters, for example, the severity of symptoms, by administration of the composition according to the present invention.

Hereinafter, preferred examples for helping with understanding of the present invention will be suggested. However, the following examples are provided only so that the present invention may be more easily understood, and the content of the present invention is not limited by the following examples.

### Example 1. Confirmation of expression of LPS according to age in Alzheimer's dementia mouse model

### 1-1. Experimental animal model

3-, 5.5-, and 11-month-old 5XFAD Alzheimer's dementia mouse models (n = 3 to 5) were used as experimental animal models, and 11-month-old wild-type mice were used as a control.

As the 5XFAD Alzheimer's dementia mice, female heterozygous 5XFAD transgenic mice (B6SJL/mice background; 6 months old; purchased from Junbiotech, Inc., Korea) were used. 5XFAD mice were confirmed by genotype analysis and wild-type (WT) littermates of 5XFAD mice were used as a control.

### 1-2. Immunohistochemical staining

After mouse brains were removed by cardiac perfusion fixation using 4% paraformaldehyde (PFA), the removed mouse brains were fixed in a phosphate buffer solution (100 mM, pH 7.4) containing 4% PFA overnight, and then immersed in a 50 mM PBS containing a 30% sucrose solution. Then, the mouse brains were frozen, sliced into a thickness of 30 µm and stored in a storage solution (DW containing 30% ethylene glycol and 30% glycerol) at 4°C.

### Immunohistochemical staining was performed by the following method.

Sections were incubated in 1% H₂O₂ for 15 minutes, and then put into a solution in which a mouse anti-E. *coli* LPS antibody (Abcam, ab35654) was diluted 1:400, and incubated at 4°C overnight. After incubation with a biotinylated secondary antibody (diluted 1:200, Vector Laboratories, Inc., Burlingame, CA, USA) at room temperature for 1 hour, the sections were incubated with an avidin-biotin-peroxidase complex (diluted 1:100, vector) for 1 hour. Thereafter, 0.02% 3,3'-diaminobenzidine and 0.01% H₂O₂ were reacted for about 3 minutes. After each incubation stage, the sections were washed three times with PBS. Finally, the sections were mounted on gelatin-coated slides, and dehydrated while increasing an alcohol concentration and cleared in xylene.

### 1-3. Confirmation of distribution of LPS(+) cells in mouse brain

Immunohistochemical staining for lipopolysaccharide (LPS) was performed on wild-type (WT) mice and 3-, 5.5-, and 11-month-old 5XFAD mice.

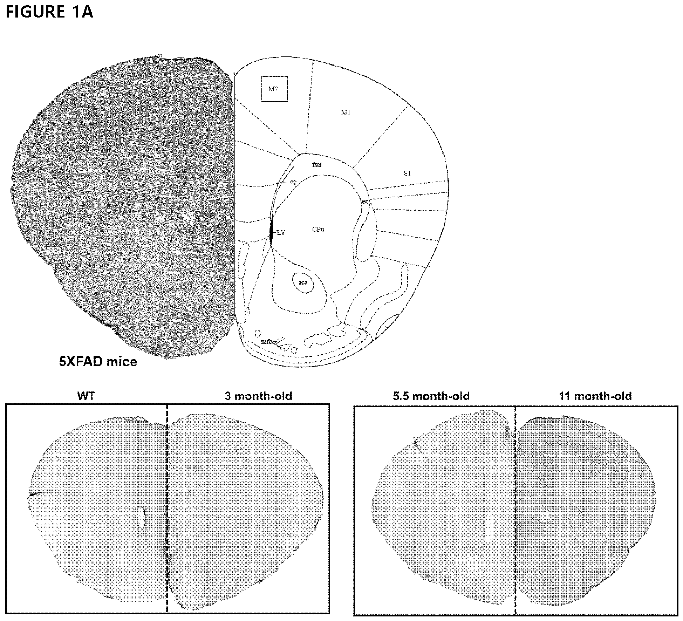

As a result of confirming the images of LPS(+) regions in the cerebral hemispheres of 5XFAD mice, as illustrated in FIG. 1A, LPS expression was not observed in the brains of wild-type (WT) mice, and LPS(+) cells and signals showed a tendency to increase with age in the brains of 5XFAD mice, and as a result of confirming the distribution of LPS(+) cells in the brains of 5XFAD mice, as illustrated in the following Table 1, it was confirmed that LPS(+) cells are abundantly distributed in the secondary motor cortex (corresponding to M2 in the top view of FIG. 1A) and mainly distributed in the polymorph layer of the dentate gyrus in the hippocampus.

**[Table 1]**

| Brain region | | | The number of LPS + cells | | |
|---|---|---|---|---|---|
| | | | 3 month-old | 5.5 month-old | **11 month-old** |
| **Cortex** | Primary motor cortex | | + | + | +++ |
| | Secondary motor cortex | | ++ | +++ | ++++ |
| | Primary somatosensory cortex | | + | ++ | +++ |
| | Secondary somatosensory cortex | | + | + | + |
| | Cingulate cortex | | + | + | ++ |
| | Frontal cortex | | + | + | ++ |
| | Parietal association cortex | | + | + | ++ |
| Hippocampus | CA1 | | - | - | - |
| | CA2 | | - | - | - |
| | CA3 | | - | - | - |
| | Dentate gyrus | Polymorph layer | + | + | ++ |
| | | Granule cel layer | - | - | - |
| | | Molecular layer | + | + | + |

### 1-4. Confirmation of LPS(+) cells in secondary motor cortex (M2) of mouse brain

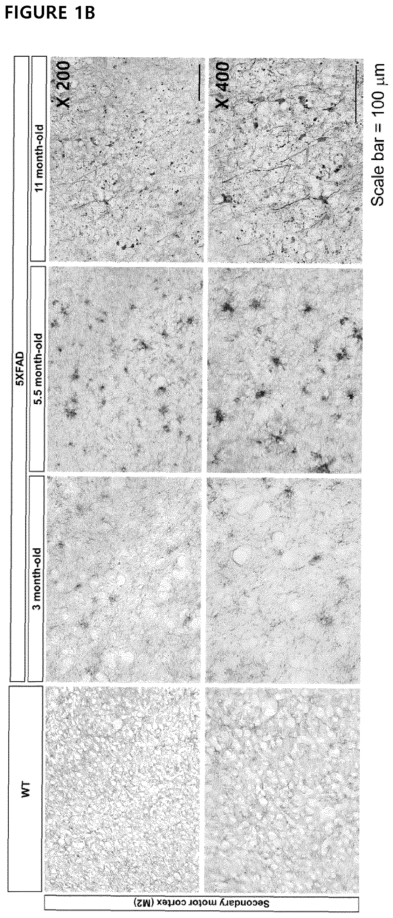

As a result of performing immunohistochemical staining in order to confirm the expression of LPS in the secondary motor cortex (M2) of the brains of 11-month-old wild-type mice and 3-, 5.5-, and 11-month-old 5XFAD mice, as illustrated in FIG. 1B, a tendency for LPS(+) cells to increase in 5XFAD mice was confirmed compared to wild-type mice, and it was confirmed that LPS(+) cells exhibited a tendency to increase with age in 5XFAD mice.

In this case, it was confirmed that LPS was localized around the nucleus (perinuclear localization), and cells expressing LPS varied depending on the age of 5XFAD mice. In the case of 3- and 5.5-month-old 5XFAD mice, LPS is presumed to be expressed mainly in microglia, and in the case of 11-month-old 5XFAD mice, the expression of LPS could also be confirmed in neurons.

### 1-5. Confirmation of LPS(+) cells in hippocampus of mouse brain

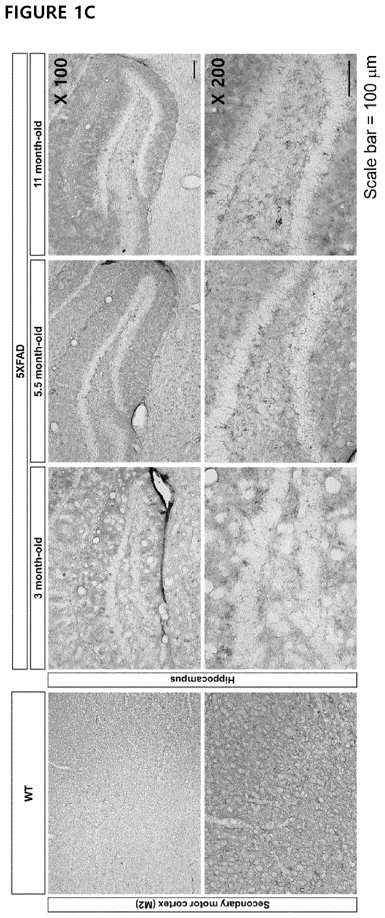

As a result of performing immunohistochemical staining in order to confirm the expression of LPS in the hippocampus of 3-, 5.5-, and 11-month-old 5XFAD mice, as illustrated in FIG. 1C, it was observed that LPS(+) cells showed a tendency to increase with age in the hippocampus of 5XFAD mice compared to wild-type mice, and it was confirmed that LPS(+) cells were mainly expressed in the polymorph layer of the hippocampal dentate gyrus.

### Example 2. Confirmation of LPS levels in Alzheimer's patient samples

LPS levels in samples of Alzheimer's patients at each clinical stage were confirmed by a limulus amebocyte lysate (LAL) test.

Specifically, the samples were divided into four groups: a normal group (n=10), asymptomatic patients (n=10), patients with premonitory symptoms of Alzheimer's dementia (n=10), and patients with Alzheimer's dementia (n=10), and then LPS levels in their blood and cerebrospinal fluid (CSF) samples were measured.

The LPS levels were measured using the LAL test, the reagents and plates to be used were prepared in advance at room temperature, and after a heat block was prepared at 37°C, 10 µl of each sample was put into a 96-well plate. Then, 10 µl of LAL reagent was added thereto, the plate was placed on its bottom and shaken, and after confirming that the resulting mixture was well mixed, the mixture was reacted at 37°C for 10 minutes. Thereafter, 20 µl of a substrate was added to each well, the well was placed on its bottom, shaken and reacted at 37°C for 6 minutes, and 20 µl of a stop solution was added to each well and shaken lightly, and then absorbance was measured at 405 nm.

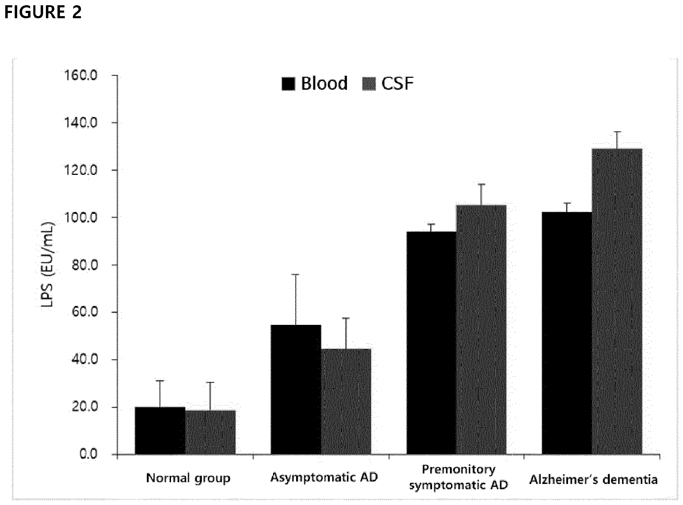

As a result, the LPS levels shown in each sample are as shown in the following Table 2 (blood samples), Table 3 (cerebrospinal fluid samples) and FIG 2.

**[Table 2]**

| EU/mL | Mean | Standard error |
|---|---|---|
| Normal group | 20.199 | 10.813 |
| Asymptomatic AD | 54.631 | 21.128 |
| Premonitory symptomatic AD | 94.015 | 3.235 |
| Alzheimer's dementia | 102.392 | 3.612 |

**[Table 3]**

| EU/mL | Mean | Standard error |
|---|---|---|
| Normal group | 18.565 | 11.806 |
| Asymptomatic AD | 44.498 | 13.039 |
| Premonitory symptomatic AD | 105.120 | 8.998 |
| Alzheimer's dementia | 128.995 | 7.303 |

As described above, as Alzheimer's dementia progressed, higher LPS concentrations in the blood and cerebrospinal fluid (CSF) were measured, confirming a high correlation between LPS and Alzheimer's dementia. Thus, in the treatment of Alzheimer's dementia, it was expected that peptides that bind well with LPS would be effective.

### Example 3: Preparation of peptide

FP1 (KLGVEAKRYLD, SEQ ID NO: 3) was denoted as wild type (WT), and FP3 (KLGVEAKRYLR, SEQ ID NO: 4) peptide with a point mutation in WT was prepared. To enhance the interaction with LPS in the residue sequence of FP3, an FP13-NH₂ (KLRRYLR-NH₂, SEQ ID NO: 5) peptide was designed based on an FP3 and LPS binding model, and the peptide of the present invention (KLRVKLRRYLR-NH₂ (AcOH), SEQ ID NO: 1, hereinafter referred to as DD-S052) was designed by additionally introducing a non-natural amino acid and an amino acid isomer (allomeric D-type amino acid (all d-form)). In addition, an N-terminal PEGylated peptide (EG-KLRVKLRRYLR-NH₂ (AcOH), SEQ ID NO: 2, hereinafter referred to as DD-S052P) was additionally designed, synthesized by ANYGEN Co., Ltd. and then provided.

(AcOH) in the peptide DD-S052 of the present invention means that trifluoroacetic acid (TFA) at the end of the ninth amino acid from the N-terminus of the peptide was substituted with an acetate salt

### Example 4. Effects of peptides on LPS-mediated cytokine production

Microglial cell line BV-2 cells were co-treated with 20 µg/ml of peptide (DD-S052) according to the invention and 2 ng/ml of LPS and cultured for 6 hours. Subsequently, the mRNA expression levels of proinflammatory cytokines IL-1β and TNF-α were measured using quantitative real-time polymerase chain reaction (PCR).

Specifically, total RNA was extracted from BV-2 cells using an RNA purification kit (Gene All, Seoul, Korea) according to the manufacturer's protocol. cDNA was synthesized using the Revert Aid First Strand cDNA Synthesis Kit (Thermo Scientific, Waltham, MA, USA) and qRT-PCR analysis was performed using the SYBR Green PCR Master Mix (Thermo Scientific, Waltham, MA, USA). The gene-specific primer sequences for IL-1β and TNF-α are shown in the following Table 4, and the mRNA levels of IL-1β and TNF-α were calculated relative to the control sample amount.

**[Table 4]**

| Primer | | Sequence | SEQ ID NO |
|---|---|---|---|
| IL-1β | Forward | 5'-ccttccaggatgaggacatga-3' | 6 |
| | Reverse | 5'-tgagtcacagaggatgggctc-3' | 7 |
| TNF-α | Forward | 5'-tcgtagcaaaccaccaagtg-3' | 8 |
| | Reverse | 5'-atatagcaaatcggctgacg-3' | 9 |

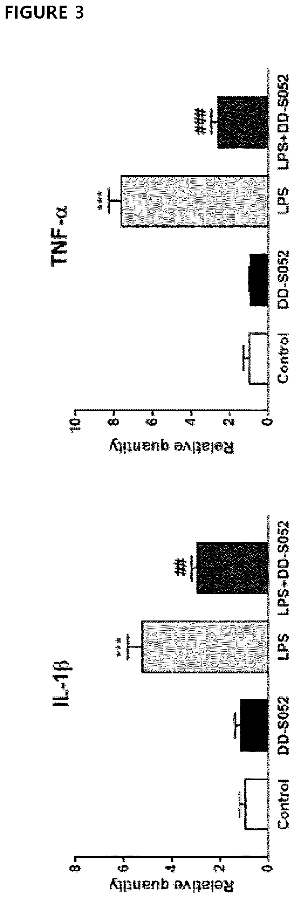

As a result, as illustrated in FIG. 3, it was confirmed that DD-S052 treatment suppressed the mRNA expression of IL-1β and TNF-α induced by LPS in BV-2 cells.

Data represents mean ± SEM, *** indicates p<0.001 for control vs. LPS-treated BV-2 cells, and ## and ### indicate p<0.01 and p<0.001 in LPS-treated BV-2 cells vs. LPS+DD-S052-treated BV-2 cells, respectively.

### Example 5. Effect of peptides on LPS-induced neuroinflammation in brains of wild-type mice

### 5-1. Effect of peptides on microgliosis induced by LPS in hippocampus of brains of wild-type mice

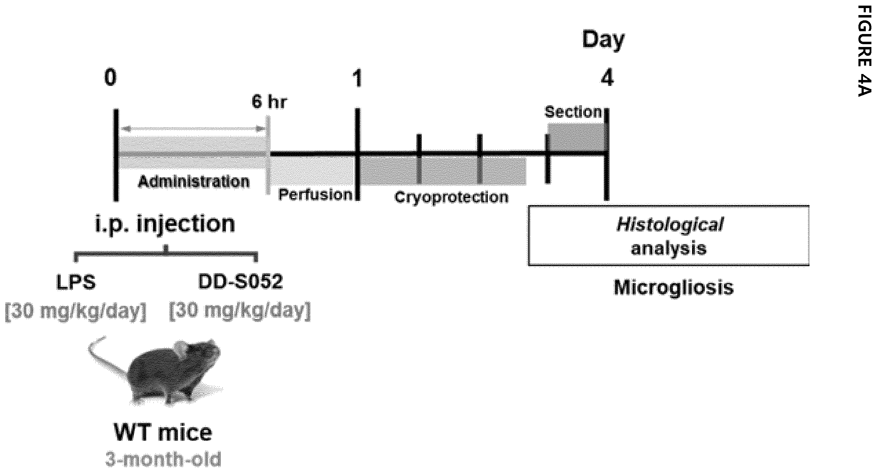

Experiments were designed as illustrated in FIG. 4A to confirm microglia in the brains of wild-type mice, and immunohistochemical staining was performed using a goat-anti Iba-1 antibody (Ab5076,1 :500) against Iba-1 which is a marker for microglia. Three-month-old wild-type mice were intraperitoneally injected (i.p.) with LPS (30 mg/kg/day) + vehicle (Hartmann dex) or DD-S052 (30 mg/kg/day) + LPS (30 mg/kg/day), and sacrificed 6 hours after the last injection of LPS. In this case, LPS and DD-S052 were loaded in separate syringes and injected separately into the right and left abdomen, respectively, and LPS was administered, and then DD-S052 was administered approximately 1 minute later. The half-life of LPS in mouse blood was approximately 2 to 4 minutes. 6 hours after administration of LPS and DD-S052, mouse brains were removed by cardiac perfusion fixation (4% PFA). Thereafter, the removed mouse brain was secondarily fixed in 4% PFA for 20 hours, and then immersed in a 30% sucrose solution. Subsequently, the mouse brains were frozen and sliced into a thickness of 30 µm.

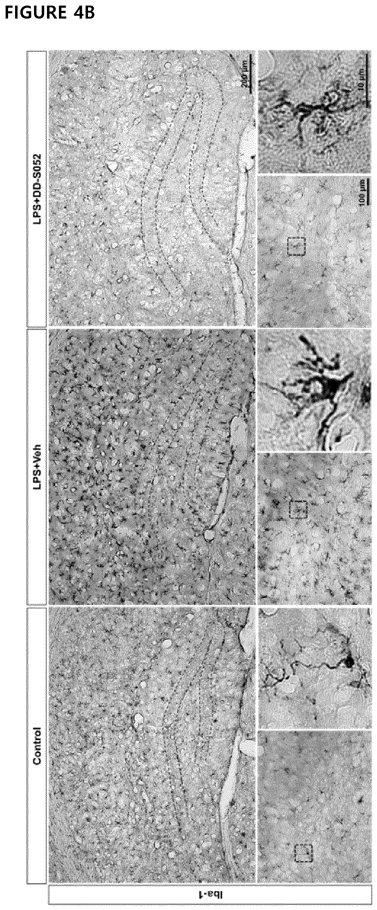

Thereafter, immunohistochemical staining for Iba-1 was performed by the method described in Example 1-2 in order to confirm microglia in the mouse hippocampus, and the results are shown in FIG. 4B. The enlarged images in FIG. 4B show that activated microglia were changed from a ramified shape to an amoeboid shape in control mice (vehicle (PBS) + vehicle (Hartmann dex) and LPS + vehicle (Hartmann dex)-treated mice, respectively. In contrast, the microglial morphology of LPS+DD-S052-treated mice showed a tendency to change from an amoeboid shape to a ramified shape.

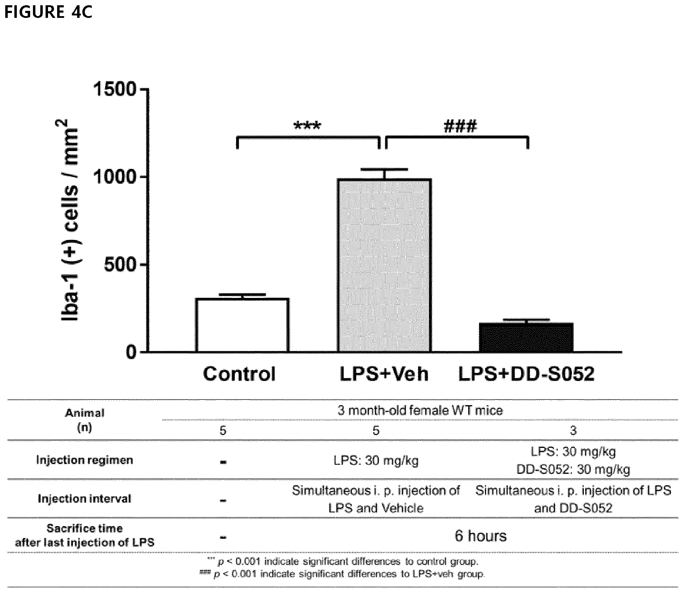

In addition, when the number of Iba-1-positive cells was quantified as illustrated in FIG. 4C, the number of Iba-1-positive cells in the hippocampus of LPS + vehicle (Hartmann dex)-treated mice was significantly increased compared to control mice, whereas the number of Iba-1-positive cells was remarkably decreased in LPS + DD-S052-treated mice compared to LPS + vehicle (Hartmann dex)-treated mice.

Data represents mean ± SEM, *** indicates a significant difference for the control (p<0.001), and ### indicates a significant difference for LPS + vehicle group (p<0.001). Scale bar = 200 µm (hippocampus), 100 µm (dentate gyrus), and 10 µm (enlarged image of microglia).

### 5-2. Effect of peptides on LPS-induced microgliosis in the substantia nigra pars compacta in wild-type mouse brain

Microglia were confirmed in the substantia nigra pars compacta (SNc) by performing immunohistochemical staining for Iba-1. Wild-type mice were treated with LPS and DD-S052 in the same manner as in Example 5-1.

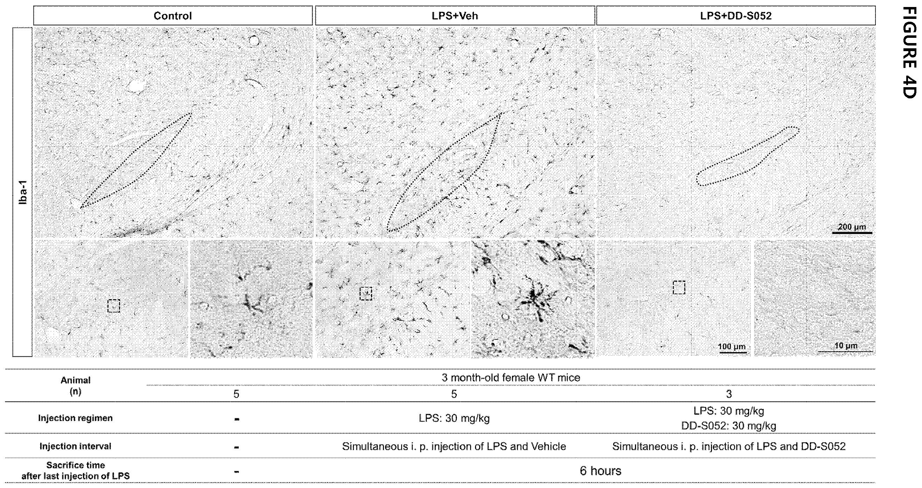

The results of performing immunohistochemical staining to confirm microglia in the substantia nigra pars compacta of the brain are illustrated in FIG. 4D. The enlarged image in FIG. 4D showed that the activated microglia changed from a ramified shape to an amoeboid shape in control mice and LPS + vehicle (Hartmann dex)-treated mice, respectively. In contrast, the microglial morphology of LPS + DD-S052-treated mice showed a tendency to change from an amoeboid shape to a ramified shape. It was confirmed that although there was a tendency in which Iba-1(+) microglia is increased in the SNc of LPS + vehicle-treated mice compared to control mice, Iba-1(+) microglia tended to decrease in the SNc of LPS + DD-S052-treated mice compared to LPS + vehicle (Hartmann dex)-treated mice. Scale bar = 200 µm, 100 µm (SNc), and 10 µm (enlarged image of microglia).

### Example 6. Effect of peptides on LPS-induced neuroinflammation in brains of mice with Alzheimer's dementia

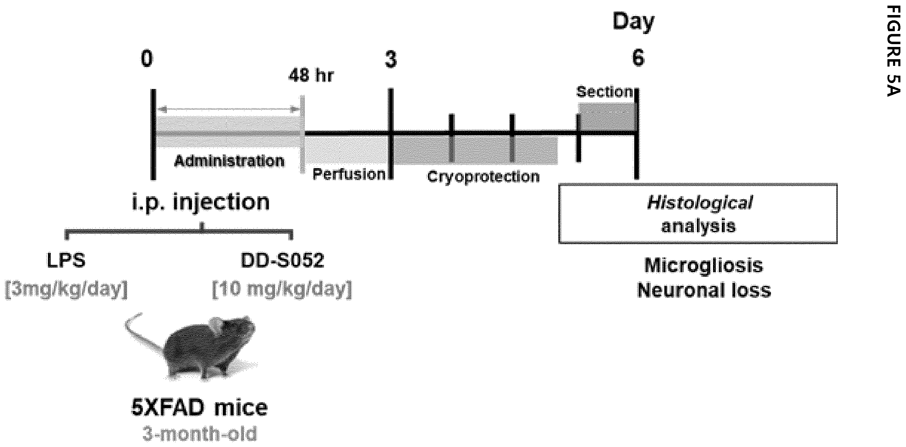

In order to confirm changes in neuroinflammation and cell death by LPS and DD-S052 in 5XFAD mice, which are an animal model of Alzheimer's dementia with beta-amyloid (Aβ) overexpression, an experiment was designed as illustrated in FIG. 5A, LPS and DD-S052 were administered to 3-month-old 5XFAD mice (n=5) that exhibited inflammation and cell death to analyze microgliosis by an immunohistochemical staining method.

Since LPS causes different degrees of inflammatory response depending on the strain of animals to be administered, LPS origin and the like, the animals were co-administered a single dose of 3 mg/kg of LPS and 10 mg/kg of DD-S052, and then sacrificed 48 hours later, 5XFAD mice were treated with 3 mg/kg of LPS by referencing a document which confirms the mortality rate by treating 5XFAD mice with 3 mg/kg of LPS (Barton, S. M.; Janve, V A.; McClure, R.; Anderson, A.; Matsubara, J. A.; Gore, J. C.; Pham, W., Lipopolysaccharide Induced Opening of the Blood Brain Barrier on Aging 5XFAD Mouse Model. J Alzheimers Dis 2019, 67, (2), 503-513.) and a document which confirms that microgliosis reaches its maximum value from day 2 after the administration of LPS (Kondo, S.; Kohsaka, S.; Okabe, S., Long-term changes of spine dynamics and microglia after transient peripheral immune response triggered by LPS in vivo. Mol Brain 2011, 4, 27) based on the LD₅₀ (1 to 25 mg/kg) of LPS. 48 hours after administration of LPS and DD-S052, mouse brains were removed by cardiac perfusion fixation (4% PFA). Thereafter, the removed mouse brain was secondarily fixed in 4% PFA for 20 hours, and then immersed in a 30% sucrose solution. Subsequently, the mouse brains were frozen and sliced into a thickness of 30 µm.

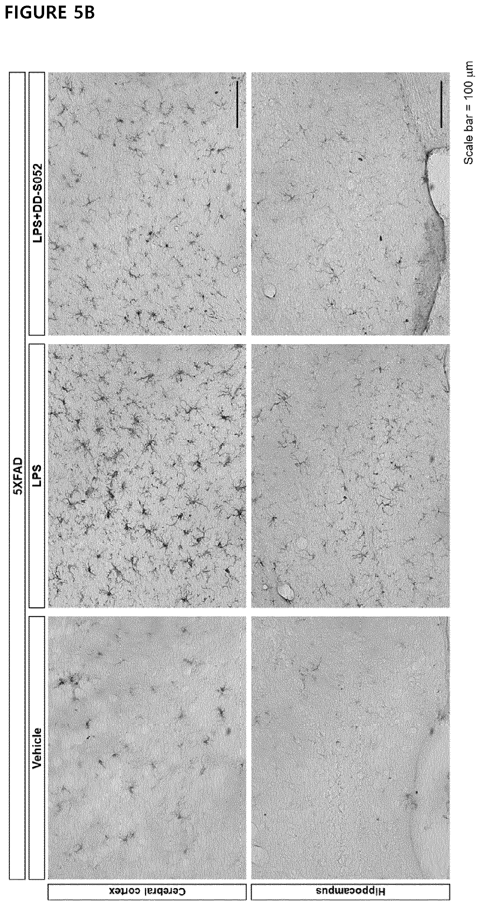

Thereafter, as a result of performing immunohistochemical staining in order to confirm microglia in the brains of 5XFAD mice, as illustrated in FIG. 5B, it was confirmed that all Iba-1(+) microglia increased during LPS + vehicle (Hartmann dex) treatment in the cerebral cortex and hippocampus, but Iba-1(+) microglia decreased during LPS + DD-S052 treatment compared to the LPS + vehicle (Hartmann dex)-treated group. Cells (positive cells) observed in dark brown are stained microglia, and changes in microglia may be confirmed by changes in the number or shape of positive cells.

### Example 7. Effect of peptides on cognitive impairment and Alzheimer's dementia-related pathology in Alzheimer's dementia mice

### 7-1. Confirmation of spontaneous alternation and total arm entry

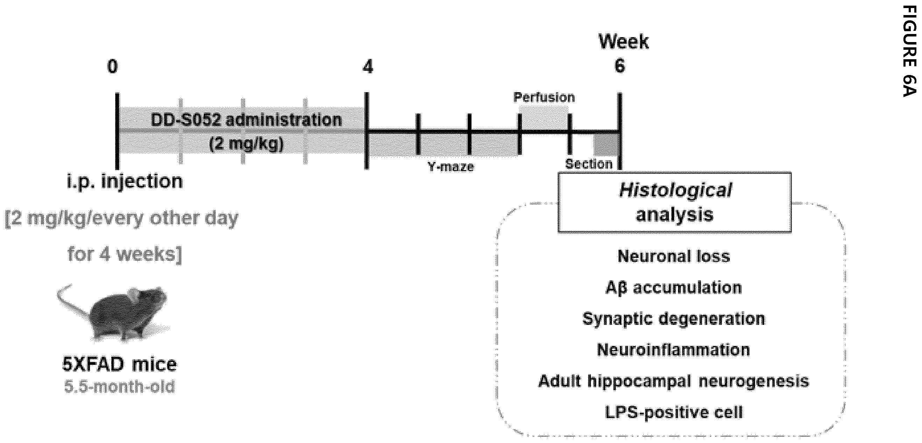

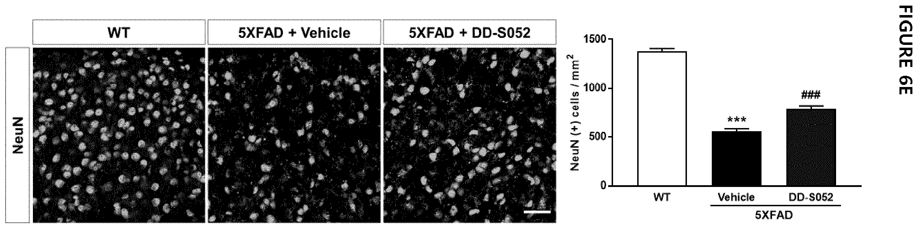

In order to confirm the effect of DD-S052 treatment on cognitive impairment and Alzheimer's dementia-related pathology in 5XFAD mice, which are an Alzheimer's dementia animal model, an experiment was designed as illustrated in FIG. 6A, and DD-S052 was administered to 5.5-month-old 5XFAD mice at 2 mg/kg every other day for 4 weeks to intraperitoneally inject a total of 30 mg/kg (n=5 to 8). Then, cognitive function experiments were performed on each mouse, and then the mouse brain was fixed by cardiac perfusion fixation. Thereafter, the removed mouse brain was secondarily fixed in a 4% PFA solution for 20 hours, and then immersed in a 30% sucrose solution. Subsequently, the mouse brains were frozen and sliced into a thickness of 30 µm.

The cognitive function experiment is a novel experiment based on the instinctive behavior of rodents with the will to explore new environments, and is an experiment to evaluate the spatial perception ability of mice to choose a path different from the one which mice chose immediately before in a maze, and scores are given by dividing spontaneous alternation (the ratio of choosing a different path from the path chosen immediately before) by the total number of passages passed (total arm entry). The total arm entry during the experiment becomes an index to evaluate whether all groups have similar motility and whether the experiment is performed normally, apart from spatial perception ability.

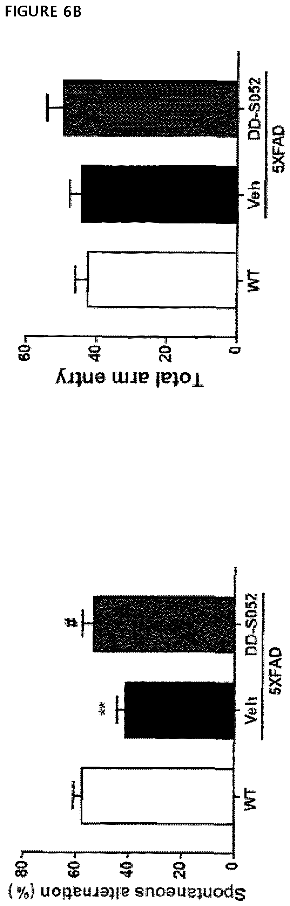

As a result of confirming cognitive impairment in the DD-S052-treated 5XFAD mice, vehicle (Hartmann dex)-treated 5XFAD mice (n=8), and vehicle (Hartmann dex)-treated wild-type mice (n=5), as illustrated in FIG. 6B, it was confirmed that the spontaneous alternation was decreased in the vehicle-treated 5XFAD mice (n = 8) compared to the wild-type mice, whereas the spontaneous alternation was increased in the DD-S052-treated 5XFAD mice compared to the vehicle-treated 5XFAD mice.

Furthermore, it was confirmed that during an 8-minute session, the total arm entry was increased in the DD-S052-treated 5XFAD mice compared to the vehicle-treated 5XFAD.

From the foregoing, it could be seen that there was a tendency in which the total arm entry was increased in the vehicle and DD-S052-treated mice, but the populations in the three groups had similar motility and the behavioral experiment was normally performed because there is no significance between groups.

### 7-2. Effect on Alzheimer's dementia-related pathology

The effects of DD-S052 on beta-amyloid accumulation, microgliosis, and neuronal loss were confirmed using an immunofluorescence staining method.

Specifically, in the case of immunofluorescence labeling, 4 to 5 coronal sections were obtained at subventricular zone (1.18 and 0.02 mm from bregma) and hippocampal formation (-1.58 and -3.80 mm from bregma) levels based on Paxinos and Franklin's "The Mouse Brain in Stereotaxic Coordinates." Brain tissue was washed with PBS using the free-floating method in a Titramax 101 orbital shaker (Heidolph, Schwabach, Germany) and sections were incubated with PBS including 0.3% Triton X-100, 0.5 mg/mL bovine serum albumin (BSA) and primary antibodies as follows at 4°C overnight: mouse anti-4G8 antibody (1:2,000; Cat. #800701, BioLegend, USA), mouse anti-NeuN antibody (1:100; Cat. #MAB377, Merck KGaA, Darmstadt, Germany), and goat anti-Iba-1 antibody (1:500; Cat. #ab5076, Abcam, Cambridge, UK).

Before being incubated with the anti-4G8 antibody, brain sections were incubated with 70% formic acid for antigen retrieval for 20 minutes, washed three times with PBS for 5 minutes each, and then incubated with a secondary antibody at room temperature for 50 minutes, and fluorescence signals were observed. As the secondary antibodies, the following antibodies were used: donkey Alexa 488-conjugated anti-mouse IgG (1:200; Cat. #A21202, Thermo Fisher Scientific Inc.), donkey Alexa 488-conjugated anti-goat IgG (1:200; Cat. #A11055, Thermo Fisher Scientific Inc.), and donkey Alexa 594-conjugated anti-rat IgG (1:200; Cat. #A21209, Thermo Fisher Scientific Inc.). All antibodies were diluted with PBS including 0.3% Triton X-100.

### 7-2-1. Effect on beta-amyloid accumulation

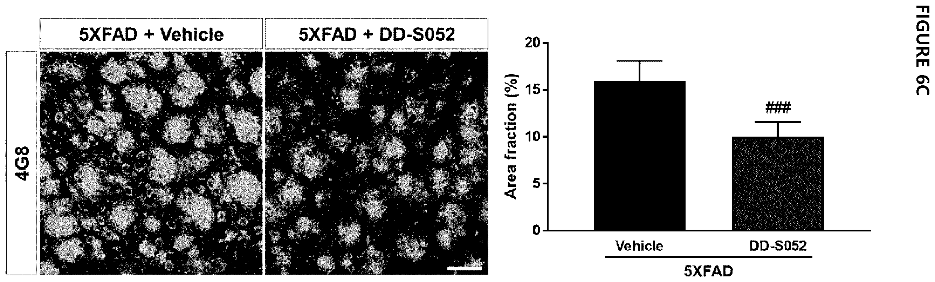

As a result of confirming beta-amyloid accumulation after administration of DD-S052 in 5XFAD mice, which are an Alzheimer's dementia animal model, as illustrated in FIG. 6C, since it was confirmed that the 4G8(+) region, which is a beta-amyloid-specific antibody, was reduced by the administration of DD-S052, it was could be seen that DD-S052 exhibited an effect of suppressing beta-amyloid accumulation.

### 7-2-2. Effect on microgliosis

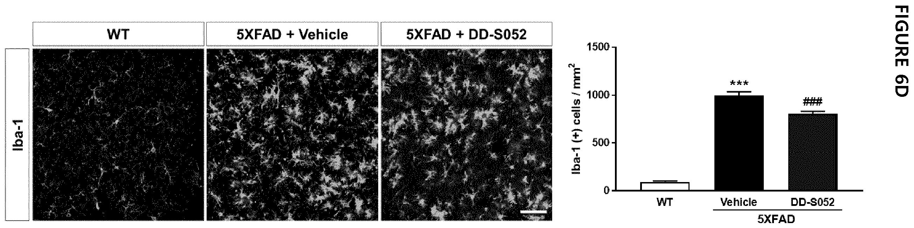

As a result of confirming the effect of DD-S052 on microgliosis in 5XFAD mice, which is an Alzheimer's dementia animal model, as illustrated in FIG. 6D, since it was confirmed that the number of Iba-1(+) cells was reduced by the administration of DD-S052 in 5XFAD mice, it could be seen that DD-S052 exhibited an effect of suppressing microgliosis.

### 7-2-3. Effect on neuronal loss

As a result of confirming the effect of DD-S052 on neuronal loss in 5XFAD mice, which is an Alzheimer's dementia animal model, as illustrated in FIG. 6D, since it was confirmed that the number of NeuN(+) cells was increased by the administration of DD-S052, it could be seen that DD-S052 exhibited an effect of suppressing neuronal loss.

### Example 8. Tau protein aggregation suppression effect of DD-5052 and PEGylated DD-5052

Thioflavin T (ThT) assay was performed to investigate the effect of DD-S052 and PEGylated DD-S052 on tau aggregation. ThT is known to bind to β-sheet-rich protein aggregates to produce fluorescence. Synthetic tau K18 was dissolved in PBS (pH 7.4) in order to induce tau aggregation. The polymerization of tau proteins was induced by preparing a solution including PBS (pH 7.4), 0.5 mg/mL tau K18, 0.1 mg/mL heparin and 100 µM DTT. DD-S052 at various concentrations (10, 20, 40, 100, and 1000 µg/mL), a 100 µM methylene blue (MB) solution (positive control), and PEGylated DD-S052 were incubated together with an aggregation mixture for 21 hours. After incubation, a ThT solution (15 µM in a 50 mM glycine buffer, pH 8.9) was added to a 96-well plate containing the sample and the aggregation mixture, and the resulting mixture was incubated for 3 hours. The fluorescence intensity of ThT was measured using a SpectraMax iD3 Multi-Mode Microplate Reader (Molecular Devices, San Jose, CA, USA) at excitation and emission wavelengths of 440 and 484 nm and quantified.

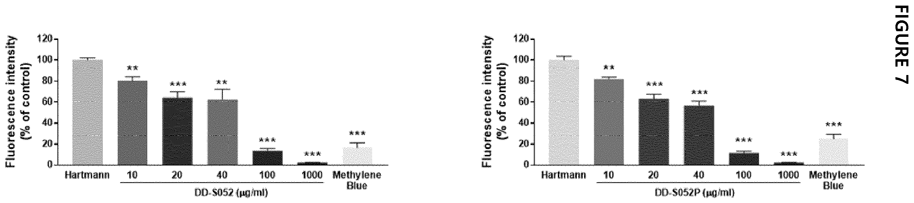

As a result of confirming the effect of DD-S052 and PEGylated DD-S052 on tau K18, as illustrated in FIG. 7, it was confirmed that the fluorescence intensity of thioflavin T (ThT), which is a fluorescent dye, was reduced in DD-S052 (left view) and PEGylated DD-S052 (DD-S052P). Statistical significance was determined by one-way analysis followed by the Tukey's multiple comparison test (*p<0.05, **p<0.01, ***p<0.001).

### Example 9. Confirmation of DD-5052 penetration through blood-brain barrier

### 9-1. Ex vivo blood-brain barrier permeation test

In order to confirm whether the peptide DD-S052 of the present invention passes through the blood-brain barrier (BBB), an artificial membrane permeation test simulating the blood-brain barrier was performed using a parallel artificial membrane permeability assay kit (PAMPA-096; BioAssay Systems).

Analysis conditions are shown in the following Table 5, and analysis was performed by liquid chromatography-mass spectrometry (LC-MS).

**[Table 5]**

| | |
|---|---|
| Membrane coating | 4% Leptin |
| Incubiton time | 18 hr, 64800s |
| Incubation temperature | room temperature |
| Buffer | PBS (Phosphate-buffered saline) |

### 9-2. Quantitative analysis

Quantitative analysis was performed using electrospray ionization coupled to Fourier transform ion cyclotron resonance mass spectrometry (ESI-FT-ICR-MS), and the analysis conditions are shown in the following Table 6.

**[Table 6]**

| | |
|---|---|
| Injection Volume | 150 µl |
| Size | 1 M |
| Avg Scans | 16 |
| Accum (s) | 0.200 |
| Nebulizing Gas Flow | 1.2bar |
| Dry Gas | 4.0 |
| Dry Temp | 200 °C |
| Colision Voltage | -3.0 V |
| DC extract bias | 1.3 |
| Time of flight | 0.9 ms |
| Frequency | 4 MHz |
| RF Amplitude | 350.0 Vpp |
| Capillary exit | 220.0 V |
| Deflector plate | 220.0 V |
| Gas flow | 30.0% |
| Transfer exit lens | -20.0V |
| Analyzer entrance | -20.0V |
| Side kick | 3.0V |
| Side kick offset | -1.6V |
| Front trap plate | 1.500V |
| Back trap plate | 1.500V |
| Back trap plate Quench | -22.0V |
| Total experiment time | 10.0682 see |
| Flow rate | 12.0 ul/h |
| Calibration ESI | TunnmgMix |

### 9-3. Analysis criteria

The intensity value of a peak corresponding to the test material was used according to the Log *Pe* function described in an existing document (Evaluation of the reproducibility of Parallel Artificial Membrane Permeation Assays PAMPA, Sigma Aldrich), and the value calculated by the Log *Pe* function was used to predict the degree of permeation by referencing an existing document (Brian.J et al. Predicting a Drug's Membrane Permeability: A Computational Model Validated With in Vitro Permeability Assay Data. J Phys Chem B. 2017 May 25;121(20):5228-5237).

### 9-4. Test results

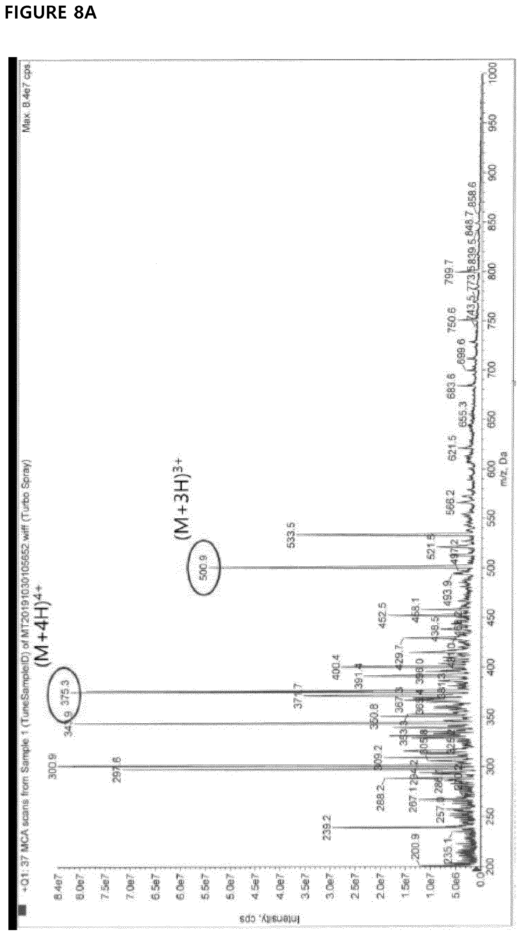

In consideration of the analytical sensitivity and reproducibility in the DD-S052 peptide analysis results, the peaks at 375.3 and 500.9 m/z were selected and analyzed as illustrated in FIG. 8A, and 100 pg/ml DD-S052 peptide was added for the artificial membrane permeation test.

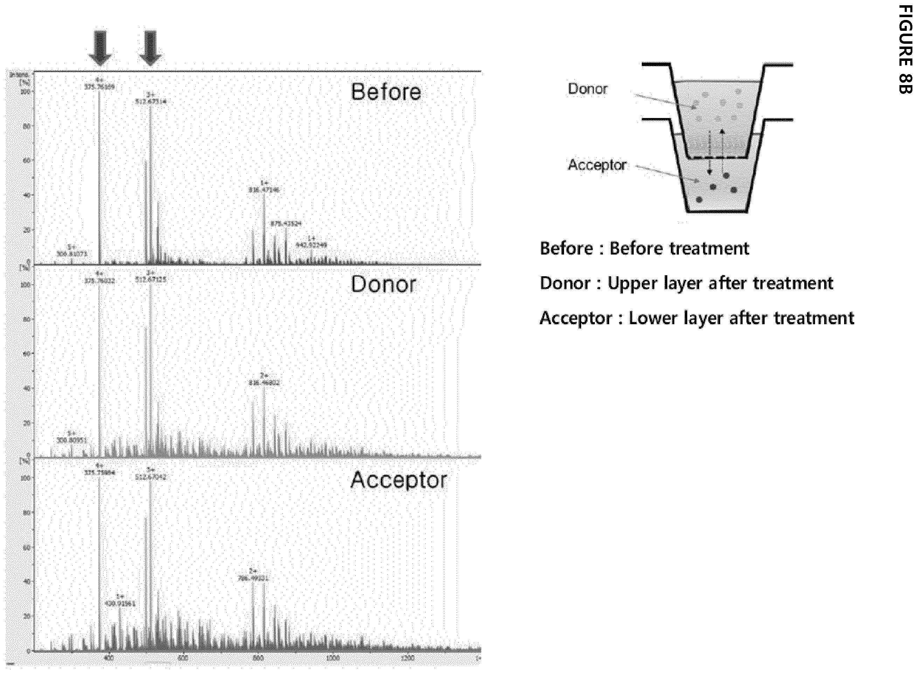

As a result of analyzing the artificial membrane permeation test results by liquid-chromatography-mass spectrometry (LC-MS), as illustrated in FIG. 8B, after DD-S052 treatment, the peaks in the upper and lower layers of the artificial membrane were similar, and as a result of quantifying and analyzing the intensity of the corresponding peak in mass spectrometry, the intensity values at 375 m/z and 512 m/z are shown in the following Tables 7 and 8.

**[Table 7]**

| | |
|---|---|
| Before | 1829478400 |
| Donor | 909677952 |
| Acceptor | 717305173 |
| Log*Pe* | -5.40519559 |

**[Table 8]**

| | |
|---|---|
| Before | 16 67509760 |
| Donor | 867968256 |
| Acceptor | 717488192 |
| Log*Pe* | -5.362403774 |

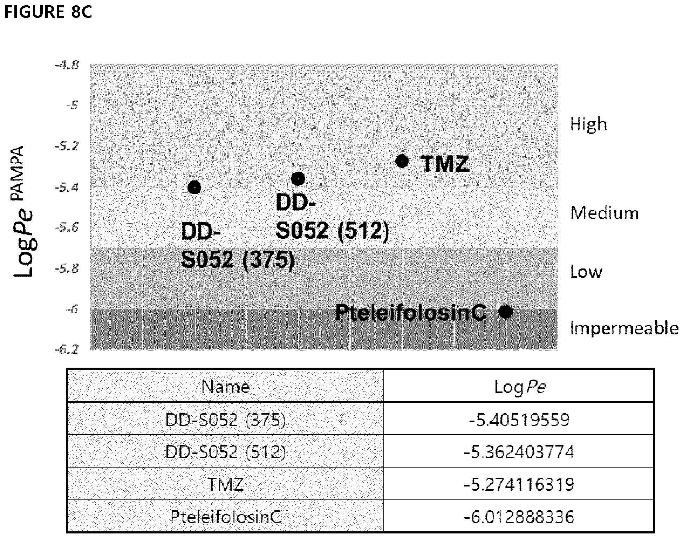

Based on the above results, the degree of permeation is classified according to the range and shown in FIG. 8C. As a control, temozolomide (TMZ) and PteleifilisinC were used. As illustrated in FIG. 8C, it was confirmed that the blood-brain barrier permeability of DD-S052 had a median log *Pe* value.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described Examples are illustrative only in all aspects and are not restrictive.

### [industrial Applicability]

A peptide or a salt substituent thereof according to the present invention exhibits effects such as suppression of LPS-mediated cytokine production, suppression of neuroinflammation, amelioration of cognitive impairment, suppression of beta amyloid or tau protein aggregation, and suppression of neuronal loss. The polypeptide or the salt substituent thereof is expected to be usefully used for preventing or treating Alzheimer's dementia.

## Claims

1. A pharmaceutical composition for preventing or treating Alzheimer's dementia, comprising a polypeptide represented by the following sequence general formula or a salt substitute thereof as an active ingredient:
[General Formula] Ln-X1-L-X2-V-X3-X4-X5-R-X6-L-X7
wherein, in General Formula,
n is 0 or 1;
L is leucine;
V is valine;
R is arginine;
X1 is lysine (K) or arginine (R);
X2 is glycine (G) or arginine (R);
X3 is glutamic acid (E) or lysine (K);
X4 is alanine (A) or leucine (L);
X5 is lysine (K), arginine (R) or leucine (L);
X6 is tyrosine (Y), alanine (A), tryptophan (W), lysine (K) or aspartic acid (D); and
X7 is aspartic acid (D) or arginine (R),
provided that in General Formula, a polypeptide represented by a sequence of K-L-G-V-E-A-K-R-Y-L-D is excluded.

2. The composition of claim 1, wherein the polypeptide is a polypeptide consisting of the following amino acids:
in General Formula,
n is 0;
X1 is lysine (K);
X2 is arginine (R);
X3 is lysine (K);
X4 is leucine (L);
X5 is arginine (R);
X6 is tyrosine (Y), and
X7 is arginine (R).

3. The composition of claim 1, wherein the polypeptide is an L-form, a D-form, a peptidomimetic comprising a peptoid, or a non-natural amino acid.

4. The composition of claim 1, wherein an end of the polypeptide is alkylated, PEGylated, or amidated.

5. The composition of claim 1, wherein the polypeptide has an amine group (NH₂) added to the C-terminus.

6. The composition of claim 1, wherein the salt substitute of the polypeptide is an acetate salt substitute.

7. The composition of claim 1, wherein the polypeptide or the salt substitute thereof has one or more characteristics of the following characteristics:
suppression of lipopolysaccharide (LPS)-mediated cytokine production;
suppression of neuroinflammation;
amelioration of cognitive impairment;
suppression of beta-amyloid or tau protein aggregation; and
suppression of neuronal loss.

8. The composition of claim 1, wherein the polypeptide or the salt substitute thereof passes through the blood-brain barrier.

9. A food composition for preventing or ameliorating Alzheimer's dementia, comprising a polypeptide represented by the following sequence general formula or a salt substitute thereof as an active ingredient:
[General Formula] Ln-X1-L-X2-V-X3-X4-X5-R-X6-L-X7
wherein, in General Formula,
n is 0 or 1;
L is leucine;
V is valine;
R is arginine;
X1 is lysine (K) or arginine (R);
X2 is glycine (G) or arginine (R);
X3 is glutamic acid (E) or lysine (K);
X4 is alanine (A) or leucine (L);
X5 is lysine (K), arginine (R) or leucine (L);
X6 is tyrosine (Y), alanine (A), tryptophan (W), lysine (K) or aspartic acid (D); and
X7 is aspartic acid (D) or arginine (R),
provided that in General Formula, a polypeptide represented by a sequence of K-L-G-V-E-A-K-R-Y-L-D is excluded.

10. The composition of claim 9, wherein the polypeptide is a polypeptide consisting of the following amino acids:
in General Formula,
n is 0;
X1 is lysine (K);
X2 is arginine (R);
X3 is lysine (K);
X4 is leucine (L);
X5 is arginine (R);
X6 is tyrosine (Y), and
X7 is arginine (R).

11. A method for preventing or treating Alzheimer's dementia, the method comprising administering a composition comprising the polypeptide or the salt substitute thereof of claim 1 as an active ingredient to a subject in need.

12. A use of a composition comprising the polypeptide or the salt substitute thereof of claim 1 as an active ingredient for preventing or treating Alzheimer's dementia.

13. A use of the polypeptide or the salt substitute thereof of claim 1 for preparing a drug for treating Alzheimer's dementia.
